(19) Europäisches Patentamt
European Patent Office
Office européen des brevets

(11) Veröffentlichungsnummer: **0 293 740 B1**

(12) **EUROPÄISCHE PATENTSCHRIFT**

(45) Veröffentlichungstag der Patentschrift: **02.12.92**

(51) Int. Cl.$^5$: **C07D 207/267**, C07D 211/70

(21) Anmeldenummer: **88108357.0**

(22) Anmeldetag: **26.05.88**

Die Akte enthält technische Angaben, die nach dem Eingang der Anmeldung eingereicht wurden und die nicht in dieser Patentschrift enthalten sind.

# (54) Verfahren zur Herstellung von substituierten Lactamen.

(30) Priorität: **03.06.87 DE 3718563**

(43) Veröffentlichungstag der Anmeldung:
**07.12.88 Patentblatt 88/49**

(45) Bekanntmachung des Hinweises auf die Patenterteilung:
**02.12.92 Patentblatt 92/49**

(84) Benannte Vertragsstaaten:
**BE CH DE ES FR GB IT LI NL**

(56) Entgegenhaltungen:
**EP-A- 0 095 278**
**EP-A- 0 116 257**
**EP-A- 0 144 664**
**US-A- 3 332 938**
**US-A- 4 235 778**

**PATENT ABSTRACTS OF JAPAN, Band 2, Nr. 72 (C-78)(695), 31. Mai 1978**

**PATENT ABSTRACTS OF JAPAN, Band 2, Nr. 72 (C-78)(696), 31. Mai 1978**

**JOURNAL OF ORGANIC CHEMISTRY, Band 47, 1982, Seiten 719-723, Easton, PA, US; P. BUCHS et al.: "(E)-5-Hydroxypyrrolizidin-3-one: Versatile Synthon for the Synthesis of 5-Substituted 2-Pyrrolidones and (Z)-3-Alkylpyrrolizindes"**

**BULLETIN OF THE ACADEMY OF SCIENCES, USSR - DEVISION OF CHEMICAL SCIENCE, Nr. 10, Oktober 1964, Seiten 1745-1748, New York, US; G.I. NIKISHIN et al.: "Free-radical addition of 2-pyrrolidinone to alpha-olefins"**

(73) Patentinhaber: **BASF Aktiengesellschaft**
**Carl-Bosch-Strasse 38**
**W-6700 Ludwigshafen(DE)**

(72) Erfinder: **Wambach, Ludwig, Dr.**
**Humboldtstrasse 28**
**W-6900 Heidelberg(DE)**
Erfinder: **Fischer, Martin, Dr.**
**Elbinger Weg 1**
**W-6700 Ludwigshafen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die vorliegende Erfindung betrifft ein neues Verfahren zur Herstellung von substituierten Lactamen der allgemeinen Formeln Ia bis Ic

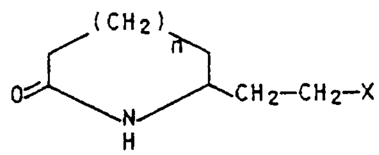
Ia

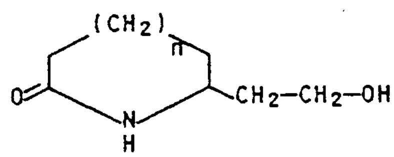
Ib

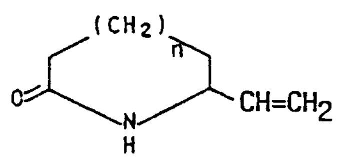
Ic

in denen n einem Wert von 0 oder 1 hat und X für eine der folgenden Gruppen

$-O-CO-R^1$
$-O-SO_2-R^1$
$-CO-R^2$
$-CO-O-R^3$
$-CO-NR^4R^5$

steht, wobei $R^1$ den Rest einer Carbon- bzw. Sulfonsäure bezeichnet, der 1 bis 8 C-Atome enthält, $R^2$ eine $C_1$-$C_8$-Alkylgruppe bedeutet, $R^3$ Wasserstoff oder eine Alkyl-, Aryl- oder Aralkylgruppe mit bis zu 8 C-Atomen bedeutet und $R^4$ und $R^5$ für Wasserstoff oder $C_1$-$C_4$-Alkylgruppen stehen, die auch zu einem 5- oder 6-gliedrigen Ring verbunden sein können.

Außerdem betrifft die Erfindung neue substituierte Lactame Ia, in denen der Rest X für einen Acetat-, Propionat- oder Toluylsulfonat-Rest steht.

Aus der US-A-3 332 938 ist es bekannt, Lactame II'

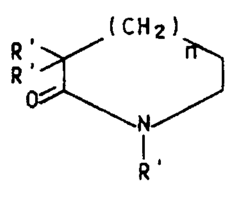
II'

R' = H, Alkyl

und α-Olefine R'' $-CH=CH_2$ mit Hilfe radikalbildender Verbindungen wie organischen Peroxiden zu substituierten Lactamen I'

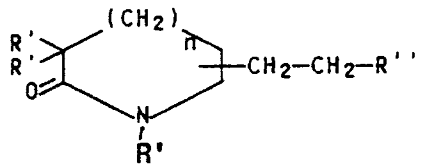 I'

R" = H, Alkyl

umzusetzen, wobei jedoch Gemische verschiedener Substitutionsprodukte anfallen. So erhält man bei der Umsetzung von Pyrrolid-2-on mit Oct-1-en etwa 25 bis 30 % 3-Octylpyrrolid-2-on und 60 bis 65 % 5-Octylpyrrolid-2-on. Über ähnliche Beobachtungen berichten G.I. Nikishin und R.I. Mustafaer in ihrer Arbeit "Free-radical addition of 2-pyrrolidone to α-olefins" (Bulletin of the Academy of Sciences, USSR, Div. of Chem. Science, 1964, Seiten 1745-48).

Weiterhin sind aus der EP-A-0 144 664 die Vinyllactame Ic

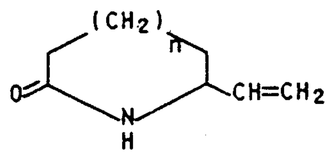 Ic

bekannt, die nach dem dort beschriebenen Verfahren aber nur auf eine äußerst umständliche Weise über die entsprechenden ω-Cyanomethyllactame erhältlich sind.

Da die Ringspaltung von Ic zu einer Gruppe wichtiger Arzneimittel führt, darunter zu dem Transaminase-Inhibitor Ic' [näheres siehe US-A 4 235 778 und 3 959 356)

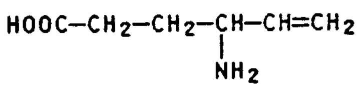 Ic'

lag der Erfindung die spezielle Aufgabe zugrunde, die Vinyllactame Ic besser zugänglich zu machen.

Allgemein erstreckt sich die Aufgabe auf ein wirtschaftliches und universell anwendbares Verfahren zur gezielten Einführung der Reste $-CH_2-CH_2-X$ und $-CH_2-CH_2-OH$ in die ω-Position von Lactamen II

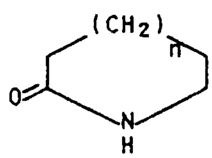 II

sowie auf die hierbei erhältlichen neuen Zwischenprodukte und solche Lactame Ia, in denen X einen Acetat-, Propionat- oder Toluylsulfonat-Rest bedeutet.

Demgemäß wurde ein Verfahren zur Herstellung von substituierten Lactamen der allgemeinen Formeln Ia bis Ic

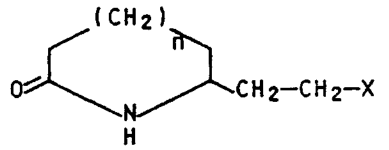

Ia

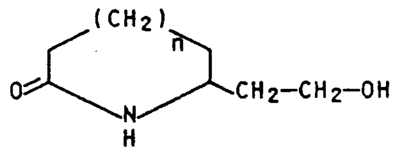

Ib

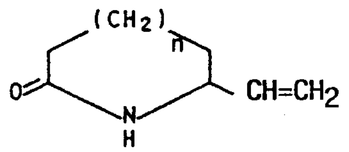

Ic

gefunden, in denen n einen Wert von 0 oder 1 hat und X für eine der folgenden Gruppen

-O-CO-$R^1$
-O-$SO_2$-$R^1$
-CO-$R^2$
-CO-O-$R^3$
-CO-$NR^4R^5$

steht, wobei $R^1$ den Rest einer Carbon- bzw. Sulfonsäure bezeichnet, der 1 bis 8 C-Atome enthält, $R^2$ eine $C_1$-$C_8$-Alkylgruppe bedeutet, $R^3$ Wasserstoff oder eine Alkyl-, Aryl- oder Aralkylgruppe mit bis zu 8 C-Atomen bedeutet und $R^4$ und $R^5$ für Wasserstoff oder $C_1$-$C_4$-Alkylgruppen stehen, die auch zu einem 5- oder 6-gliedrigen Ring verbunden sein können, welches dadurch gekennzeichnet ist, daß man

a) zur Herstellung von Ia ein Lactam der allgemeinen Formel II

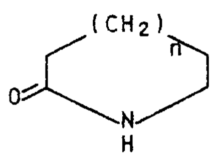

II

bei 100 bis 200 °C mit einer Vinylverbindung der allgemeinen Formel III

$CH_2 = CH\text{-}X$ III

und einer radikalbildenden Verbindung IV umsetzt,

b) zur Herstellung von Ib solche Verbindungen Ia, in denen X eine (-O-CO-$R^1$)- oder (-O-$SO_2$-$R^1$)-Gruppe bedeutet, hydrolytisch spaltet und

c) zur Herstellung von Ic aus den Verbindungen Ib oder solchen Verbindungen Ia, in denen X eine (-O-CO-$R^1$)- oder (-O-$SO_2$-$R^1$)-Gruppe bedeutet, Wasser bzw. die Säuren $R^1$-COOH oder $R^1$-$SO_3H$ thermisch eliminiert.

Unter den Ausgangsverbindungen II, dem Pyrrolid-2-on und dem Piperidin-2-on, kommt dem Pyrrolid-2-on im Hinblick auf die Herstellung der physiologisch wichtigen γ-Aminosäure Ic' besondere Bedeutung zu. Darin, daß es sich bei diesen Ausgangsmaterialien um handelsübliche billige Verbindungen handelt, liegt ein wesentlicher Vorteil des erfindungsgemäßen Verfahrens.

Als Vinylverbindungen III seien genannt:

- aliphatische Carbonsäurevinylester wie insbesondere solche von $C_2$-$C_4$-Fettsäuren, darunter vor allem

Vinylacetat und Vinylpropionat; $R^1$ kann aber auch einen aromatischen, araliphatischen oder cycloaliphatischen Rest bedeuten, der inerte Substituenten wie Halogen tragen kann;

- Sulfonsäurevinylester wie besonders Vinyltoluolsulfonat; allgemein gilt bezüglich des Restes $R^1$ das gleiche wie für die Carbonsäurevinylester;
- Vinylketone wie Vinylmethylketon und Vinylpropylketon;
- Acrylsäure und Acrylsäureester wie Methylacrylat, Ethylacrylat, Phenylacrylat und Benzylacrylat, und
- Acrylamide wie der Stammkörper dieser Verbindungsklasse, das N,N-Dimethylacrylamid und das N-Acryloylpiperidin.

Möchte man die Verbindungen Ib herstellen, empfiehlt sich als Vinylverbindung III aus wirtschaftlichen und verfahrenstechnischen Gründen besonders das Vinylacetat, und zur Herstellung von Ic kommen vor allem die in diesem Falle als Zwischenprodukte fungierenden Verbindungen Ib sowie die Umsetzungsprodukte der Lactame II mit Vinylacetat und Vinyltoluolsulfonat in Betracht.

Für den Verfahrensschritt (a) eignen sich als Radikalbildner IV alle diejenigen Peroxide und Azoverbindungen, welche man zur radikalischen Polymerisation von olefinisch ungesättigten Monomeren verwendet, also z.B. Hydroperoxide wie tert.-Butylhydroperoxid und Cumolhydroperoxid, Dialkylperoxide wie Di-tert.-butylperoxid, Diacylperoxide wie Dibenzoylperoxid, Peroxisäuren und deren Ester wie Perbenzoesäure und tert.-Butylperbenzoat und Azoverbindungen wie Azodiisobutyronitril. Besonders bevorzugt wird Di-tert.-butylperoxid. Im übrigen richtet sich die Wahl des Radikalbildners nach der Reaktionstemperatur, indem man zweckmäßigerweise einen solchen Radikalbildner verwendet, dessen Zerfallstemperatur kurz unterhalb der Reaktionstemperatur liegt.

Die Menge des Radikalbildners beträgt im allgemeinen 5 bis 20, vorzugsweise 8 bis 15 mol-% der Vinylverbindung III. Unterhalb von 5 mol-% verlangsamt sich die Reaktion zu sehr, und größere Mengen als 30 mol-% bringen keine wirtschaftlichen Vorteile mehr.

Der Bereich von 100 bis 200 °C für die Reaktionstemperatur ergibt sich einerseits daraus, daß unterhalb von 100 °C mit vermehrten Radikalkettenabbrüchen zu rechnen ist und andererseits sind oberhalb von 200 °C Nebenreaktionen zu beobachten. In der Regel arbeitet man vorzugsweise bei Temperaturen zwischen 130 und 180 °C.

Prinzipiell hat der Druck keinen Einfluß auf die Reaktion, so daß man sie vorzugsweise unter Normaldruck durchführt, jedoch kann sich ein höherer Druck - etwa bis zu 10 bar - dann empfehlen, wenn die Vinylverbindung III wie beispielsweise Vinylacetat unter den Reaktionsbedingungen flüchtig ist.

Um die Polymerisation der Vinylverbindungen III, die zur erfindungsgemäßen Umsetzung stets in Konkurrenz tritt, nach Möglichkeit zu unterdrücken, sorgt man zweckmäßigerweise dafür, daß das Lactam II während der Reaktion stets in großem Überschuß vorliegt, etwa indem man es zusammen mit dem Radikalbildner vorlegt und hierzu bei der Reaktionstemperatur allmählich die Vinylverbindung gibt. Eine andere Möglichkeit besteht darin, die Vinylverbindung und eine Lösung des Radikalbildners in II synchron, gegebenenfalls in vorgelegtes II, zusammenzuführen. Bezogen auf die gesamte Umsetzung (a) empfiehlt es sich, II und III im Molverhältnis von 2:1 bis 50:1, vorzugsweise von 5:1 bis 20:1 einzusetzen.

Die Mitverwendung von Lösungsmitteln ist an sich nicht erforderlich, da die Lactame II meistens im Überschuß eingesetzt werden und dabei gleichzeitig als Lösungs- und Verdünnungsmittel dienen. Will man trotzdem Lösungsmittel verwenden, etwa um die Vinylverbindungen III oder die Radikalbildner IV in Form von anderen Lösungen als denen in den Lactamen II einzusetzen, so eignen sich hierfür beispielsweise Chlorbenzol und die Dichlorbenzole in Mengen bis zu 500 Vol.% der zu lösenden Komponente.

Häufig ist es zweckmäßig, unter Inertgasatmosphäre zu arbeiten, um Reaktionen des Luftsauerstoffs, die durch den Radikalbildner begünstigt werden, auszuschließen.

Die Aufarbeitung der Reaktionsgemische auf die Verfahrensprodukte Ia kann wie üblich vorgenommen werden, indem man beispielsweise den überschüssigen Radikalbildner mit Reduktionsmitteln wie Eisen-(II)-salzen und Iodid-Lösungen zerstört, danach den Großteil des Lactam II sowie gegebenenfalls das Lösungsmittel abdestilliert und Ia aus dem Rückstand, eventuell nach Zusatz eines löslichkeitsvermindernden Lösungsmittels auskristallisieren läßt. In vielen Fällen ist jedoch eine rein destillative Isolierung von Ia möglich.

Die zur Herstellung von Ia erforderlichen Reaktionszeiten liegen im allgemeinen zwischen 1 und 5 Stunden. Man erhält die Verbindungen Ia in der Regel in Ausbeuten von 50 bis 85 %, bezogen auf die Vinylverbindung III. Hierbei ist hervorzuheben, daß der Anteil isomerer Verbindungen meistens vernachlässigbar gering ist.

Im Verfahrensschritt (b) geht man zur Herstellung der Verbindungen Ib von solchen Verbindungen Ia aus, in denen X eine ($-O-CO-R^1$)- oder ($-O-SO_2-R^1$)-Gruppe bedeutet, wobei man zweckmäßigerweise die Reaktionsgemische des Verfahrensschritts (a) einsetzt, denn die Reindarstellung von Ia ist meistens nicht erforderlich.

Man nimmt die hydrolytische Abspaltung zweckmäßigerweise mit einem Überschuß Wasser sowie in Gegenwart katalytischer Mengen einer Mineralsäure wie Schwefelsäure oder einem sauren Ionenaustauscher oder mit stöchiometrischen Mengen einer Mineralbase wie Natronlauge oder eines tertiären Amins wie Triethylamin, bei etwa 50 bis 120 °C wie üblich vor. Eine weitere Möglichkeit besteht darin, mit einem Überschuß Säure zu arbeiten und das Reaktionsgemisch anschließend wieder zu neutralisieren. Die Isolierung des Alkohols Ib kann wie üblich vorgenommen werden, so daß sich nähere Ausführungen hierzu erübrigen. Man erhält die ω-Hydroxyethyllactame Ib in nahezu quantitativer Ausbeute.

Im Verfahrensschritt (c) spaltet man zur Gewinnung der Verbindungen Ic das Wasser aus den Verbindungen Ib oder die Säure $R^1$-COOH bzw. $R^1$-$SO_2$-OH aus den entsprechenden Verbindungen Ia thermisch oder thermisch-katalytisch ab, indem man die Ausgangsstoffe verdampft und auf 300 bis 700 °C erhitzt. Im Falle der Dehydratisierung von Ib empfehlen sich bekannte Dehydratisierungskontakte wie Aluminiumoxid oder Zeolithe als Katalysatoren.

Man kondensiert die Spaltprodukte und arbeitet das Kondensat wie üblich, vorzugsweise destillativ, auf die ω-Vinyllactame Ic auf. Die Ausbeuten an Ic, bezogen auf das eingesetzte Ia bzw. Ib, liegen in der Regel im Bereich von 80 bis 95 %.

Das erfindungsgemäße Verfahren zeichnet sich dadurch aus, daß man von billigen Ausgangsstoffen auf denkbar einfache Weise zu den praktisch isomerenfreien ω-substifuierten Lactamen gelangt, die wertvolle Zwischenprodukte für organische Synthese, zur Herstellung physiologisch aktiver Stoffe, darunter den Trans-aminasehemmern Ic' sind.

Beispiel 1

5-(2-Acetoxy)-ethylpyrrolid-2-on (Ia/1)

Eine Mischung aus 255 g (3 mol) Pyrrolid-2-on, 258 g (3 mol) Vinylacetat und 88 g (0,6 mol) Di-tert.-butylperoxid wurde unter Stickstoffatmosphäre im Laufe von 9 Stunden allmählich zu 2550 g (30 mol) Pyrrolid-2-on gegeben, das auf 150 °C gehalten wurde. Nach weiteren 2 Stunden bei 150 °C wurde das Reaktionsgemisch destillativ auf das Verfahrensprodukt Ia/1 aufgearbeitet; Sdp.: 143-146 °C/0,15 mbar; Ausbeute 60 %.

Beispiel 2

5-(2-Methoxycarbonyl)-ethylpyrrolid-2-on (Ia/2)

Diese Verbindung wurde analog Beispiel 1 aus Pyrrolid-2-on und Methylacrylat hergestellt. Ausbeute 80 %; Sdp.: 135-138 °C/0,15 mbar; Smp.: 60-62 °C.

Beispiel 3

6-(2-Acetoxy)-ethylpiperid-2-on (Ia/3)

Diese Verbindung wurde analog Beispiel 1 aus Piperid-2-on und Vinylacetat hergestellt. Ausbeute 55 %; Sdp.: 140-143 °C/0,1 mbar.

Beispiel 4

6-(2-Hydroxy)-ethylpiperid-2-on (Ib/1)

74 g (0,4 mol) des Piperidons Ia/3 gemäß Beispiel 3 wurden mit 500 ml 20 gew.%iger Schwefelsäure 8 Stunden lang auf 85 °C erhitzt, wonach das Reaktionsgemisch mit Natronlauge neutralisiert und anschließend mit Diethylether als Extraktionsmittel versetzt wurde. Der Ether wurde von der getrockneten Etherphase abgezogen und der Rückstand wurde aus Methylacetat/Cyclohexan umkristallisiert. Ausbeute an Ib/1 75 %; Smp.: 78-80 °C.

Beispiel 5

Herstellung von 5-Vinylpyrrolid-2-on (Ic/1)

Ein Stickstoffstrom von 40 l/h, der rd. 26 g/l des Pyrrolidons Ia/1 gemäß Beispiel 1 enthielt, wurde durch einen auf 520 °C erhitzten Rohrreaktor von 45 cm Höhe und 3 cm Innendurchmesser geleitet, der mit Porzellanringen von 5 mm Durchmesser gefüllt war. Die Verweilzeit des Gases im Reaktor betrug rd. 28 sec. Nach Passieren des Reaktors wurde der Gasstrom abgekühlt, und aus der Zusammensetzung des hierbei gewonnenen Kondensates ergab sich, daß sich Ia/1 zu 70 % umgesetzt hatte, wobei die Selektivität bezüglich des Vinylpyrrolidons Ic/1 85 % betrug. Sdp.: 85-90 °C/0,1 mbar.

Beispiel 6

Herstellung von 5-(2-Carboxy)-ethylpyrrolid-2-on (Ia/4)

Diese Verbindung wurde analog Beispiel 1 aus Pyrrolid-2-on und Acrylsäure hergestellt. Ausbeute: 63 %; Smp.: 123-125 °C.

## Patentansprüche

**1.** Verfahren zur Herstellung von substituierten Lactamen der allgemeinen Formeln Ia bis Ic

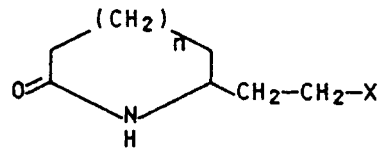

Ia

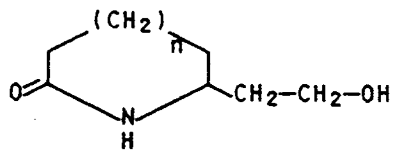

Ib

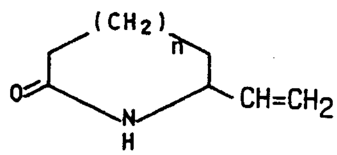

Ic

in denen n einen Wert von 0 oder 1 hat und X für eine der folgenden Gruppen

$-O-CO-R^1$
$-O-SO_2-R^1$
$-CO-R^2$
$-CO-O-R^3$
$-CO-NR^4R^5$

steht, wobei $R^1$ den Rest einer Carbon- bzw. Sulfonsäure bezeichnet, der 1 bis 8 C-Atome enthält, $R^2$ eine $C_1$-$C_8$-Alkylgruppe bedeutet, $R^3$ Wasserstoff oder eine Alkyl-, Aryl- oder Aralkylgruppe mit bis zu 8 C-Atomen bedeutet und $R^4$ und $R^5$ für Wasserstoff oder $C_1$-$C_4$-Alkylgruppen stehen, die auch zu einem 5- oder 6-gliedrigen Ring verbunden sein können, dadurch gekennzeichnet, daß man

a) zur Herstellung von Ia ein Lactam der allgemeinen Formel II

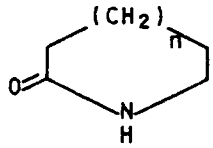

II

bei 100 bis 200 °C mit einer Vinylverbindung der allgemeinen Formel III

$CH_2 = CH\text{-}X$ III

und einer radikalbildenden Verbindung IV umsetzt,

b) zur Herstellung von Ib solche Verbindungen Ia, in denen X eine (-O-CO-$R^1$)- oder (-O-$SO_2$-$R^1$)-Gruppe bedeutet, hydrolytisch spaltet und

c) zur Herstellung von Ic aus den Verbindungen Ib oder solchen Verbindungen Ia, in denen X eine (-O-CO-$R^1$)- oder (-O-$SO_2$-$R^1$)-Gruppe bedeutet, Wasser bzw. die Sauren $R^1$-COOH oder $R^1$-$SO_3H$ thermisch oder thermisch-katalytisch eliminiert.

**2.** Substituierte Lactame der allgemeinen Formel Ia gemäß Anspruch 1, in der X für einen Acetat-, Propionat- oder Toluylsulfonat-Rest steht.

## Claims

**1.** A process for the preparation of a substituted lactam of the formula Ia, Ib or Ic

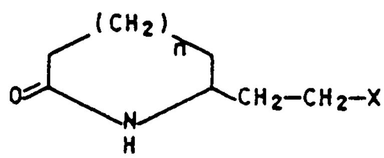

Ia

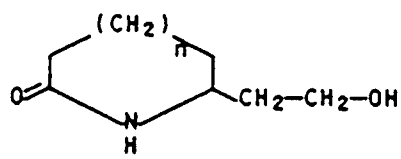

Ib

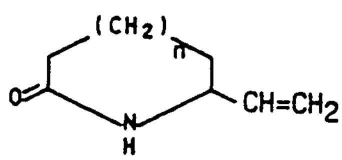

Ic

where n is 0 or 1 and X is one of the following groups

-O-CO-$R^1$
-O-$SO_2$-$R^1$
-CO-$R^2$
-CO-O-$R^3$
-CO-N$R^4R^5$

where $R^1$ is a radical of a carboxylic or sulfonic acid of 1 to 8 carbon atoms, $R^2$ is $C_1$-$C_8$-alkyl, $R^3$ is hydrogen or an alkyl, aryl or aralkyl group of not more than 8 carbon atoms and $R^4$ and $R^5$ are each hydrogen or $C_1$-$C_4$-alkyl and may furthermore be bonded to form a 5-membered or 6-membered ring, wherein

a) for the preparation of Ia, a lactam of the formula II

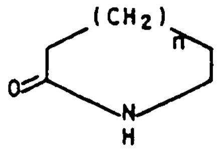

II

is reacted at from 100 to 200 ° C with a vinyl compound of the formula III

$CH_2 = CH\text{-}X$ III

and a compound IV which forms free radicals,

b) for the preparation of Ib, a compound Ia in which X is a (-O-CO-$R^1$) or (-O-$SO_2$-$R^1$) group is subjected to hydrolytic cleavage, and

c) for the preparation of Ic from a compound Ib or from a compound Ia in which x is a (-O-CO-$R^1$) or (-O-$SO_2$-$R^1$) group, water or an acid $R^1$-COOH or $R^1$-$SO_3$H is eliminated by heating in the presence or absence of a catalyst.

**2.** A substituted lactam of the formula Ia as claimed in claim 1, wherein X is acetate, propionate or toluylsulfonate.

**Revendications**

**1.** Procédé de préparation de lactames substitués des formules générales Ia à Ic

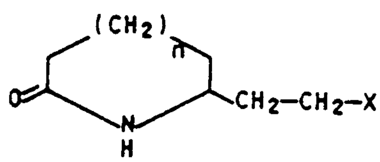

Ia

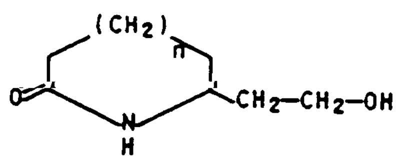

Ib

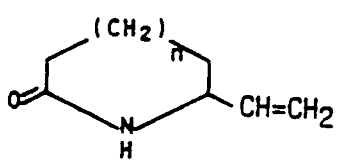

Ic

dans lesquelles n est égal à 0 ou à 1 et X représente l'un des radicaux qui suivent

-O-CO-$R^1$
-O-$SO_2$-$R^1$
-CO-$R^2$
-CO-O-$R^3$
-CO-N$R^4$$R^5$

où $R^1$ représente le reste d'un acide carboxylique ou sulfonique, qui contient de 1 à 8 atomes de carbone, $R^2$ représente un radical alkyle en $C_1$-$C_8$, $R^3$ représente un atome d'hydrogène ou un radical alkyle, aryle, aralkyle avec jusqu'à 8 atomes de carbone et $R^4$ et $R^5$ représentent des atomes d'hydrogène ou des radicaux alkyle en $C_1$-$C_4$, qui peuvent aussi être liés en un cycle pentagonal ou

hexagonal, caractérisé en ce que

a) en vue de la préparation de Ia, on fait réagir un lactame de la formule générale II

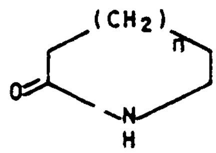

II

à une température de 100 à 200 ° C avec un composé vinylique de la formule générale III

$CH_2 = CH\text{-}X$ III

et un composé IV formateur de radicaux,

b) en vue de la préparation de Ib, on soumet à scission ou séparation hydrolytique des composés Ia) du genre de ceux dans lesquels X représente un radical ($-O-CO-R^1$) ou ($-O-SO_2-R^1$) et

c) en vue de la préparation de Ic), on élimine par voie thermique ou thermocatalytique de l'eau ou les acides $R^1$-COOH ou $R^1$-$SO_3H$ à partir des composés Ib ou de composés Ia du genre de ceux dans lesquels X représente un radical ($-O-CO-R^1$) ou ($-O-SO_2-R^1$).

**2.** Lactames substitués de la formule générale Ia selon la revendication 1, dans lesquels X représente un radical acétate, propionate ou toluylsulfonate.